# EUROPÄISCHE PATENTSCHRIFT

(11) **EP 0 775 003 B1**
(45) Veröffentlichungstag und Bekanntmachung des Hinweises auf die Patenterteilung: **14.04.1999**
(21) Anmeldenummer: 94914420.8
(22) Anmeldetag: 26.04.1994
(51) Int. Cl.: A63J 23/02, A63J 5/00, E04H 3/10, E04H 3/22

(54) **VERFAHREN UND VORRICHTUNG ZUR SYNCHRONEN DARBIETUNG VON DÜFTEN ZU VISUELLEN UND/ODER AKUSTISCHEN REIZEN**
PROCESS AND DEVICE FOR THE SYNCHRONOUS ADDITION OF ODOURS TO VISUAL AND/OR ACOUSTIC STIMULATION
PROCEDE ET DISPOSITIF PERMETTANT D'ADJOINDRE DE MANIERE SYNCHRONE LA DIFFUSION D'ODEURS A DES STIMULATIONS VISUELLES ET/OU ACOUSTIQUES

(30) Priorität: 05.05.1993 DE 4314886
(43) Veröffentlichungstag der Anmeldung: 28.05.1997
(60) Teilanmeldung: 98118174.6
(73) Patentinhaber: WITTEK, Götz-Ulrich, London W1R 5FA (GB)
(72) Erfinder: WITTEK, Götz-Ulrich, London W1R 5FA (GB)
(74) Vertreter: Diehl, Hermann, Dr. Dipl.-Phys.
(86) Internationale Anmeldenummer: EP9401314
(87) Internationale Veröffentlichungsnummer: WO9426375

(56) Entgegenhaltungen:
- CN-A- 1 059 038
- US-A- 3 795 438
- US-A- 4 603 030
- US-A- 4 629 604

## Beschreibung

Die Erfindung betrifft ein Verfahren zur synchronen Darbietung von Düften zu visuellen und/oder akustischen Reizen.

Aus der US-A-3,795,438 sind ein Verfahren und eine Vorrichtung zur Zuführung gesteuerter Mengen von Düften in einem Zuschauerraum, im allgemeinen in Verbindung mit beispielsweise einer visuellen Darstellung, bekannt. Kartuschen oder andere Aufnahmebehälter, welche Duftsubstanzen enthalten, sind derart ausgestaltet, daß diese mit einem Luftzufuhrkanal in Abhängigkeit von Markierungen auf beispielsweise einem Filmstreifen in Verbindung stehen. Daneben ist ein zweites Luftfördersystem vorgesehen, welches in der Lage ist, die Duftsubstanzen zu entfernen, bis die Menge derselben unterhalb der Wahrnehmungsgrenze liegt. Für die Zuführung eines Duftes sind Kanäle mit geeigneten Ventilen sowie Injektionsdüsen vorgesehen.

Die US-A-4,629,604 beschreibt eine Abspielvorrichtung für eine Multi-Aroma-Kartusche. Diese besteht aus einer planaren Anordnung von gleichartigen inneren Rahmenabschnitten, welche in einem äußeren Rahmen gehaltert sind. Jeder Rahmenabschnitt ist aus einem Kissen aus absorbierendem Material aufgebaut, eingespannt zwischen Rahmenteilen, deren Enden so zusammengefügt sind, daß ein zentraler Bereich übrigbleibt, welcher das Kissen freilegt. Das Kissen jedes einzelnen Rahmenabschnitts ist mit verschiedenen flüssigen Duftstoffen imprägniert. Wenn die Kartusche in einem Spalt des Gehäuses der Abspielvorrichtung eingeschoben wird, kommt die Kartusche über einer komplementären Bienenwabenstruktur zu liegen, wobei einzelne Zellen der Wabenstruktur bündig mit entsprechenden Rahmenabschnitten abschließen. Die einzelnen Zellen sind mit individuellen elektrischen Heizgeräten ausgestattet, so daß, wenn ein Heizgerät einer einzelnen ausgewählten Zelle eingeschaltet wird, die Luft in der Zelle erwärmt wird, wodurch ein Überdruck entsteht, welcher bewirkt, daß die erwärmte Luft durch den freiliegenden, zentralen Bereich des Kissens unter Verdampfung des flüssigen Aromastoffes entweicht. Der entstehende aromatisierte Dampf wird dann durch in dem Gehäuse befindliche Auslaßöffnungen an die Umgebung abgegeben. Die Auswahl der abgegebenen Aromastoffe kann manuell erfolgen oder die Aromastoffe können mit den Szenen eines Videobandes oder einer Filmvorführung synchronisiert werden.

Die Erfindung befaßt sich mit einem Verfahren zur Erhöhung der sinnlichen Wahrnehmung von visuellen und/oder akustischen Darbietungen, insbesondere in Kinos, Theatern, Konzert- und Vortragssälen sowie bei Diavorträgen, Videos, Fernsehsendungen, Hörspielen und dergleichen, wobei den Zuschauern bzw. Zuhörern synchron zur Darbietung von bestimmten visuellen und/oder akustischen Ereignissen bzw. Szenen dazu passende Düfte zugeführt werden. Ein derartiges Verfahren und zugehörige Vorrichtungen sind in der deutschen Patentanmeldung DE-A-4135796.5 des Anmelders beschrieben.

Der vorliegenden Erfindung liegt primär die Aufgabe zugrunde, ein derartiges Verfahren dahingehend weiter zu verbessern, daß die dabei verwendeten Düfte in all ihren Charakteristika noch besser wahrnehmbar sind und möglichst wenig verfälscht werden.

Diese Aufgabe wird erfindungsgemäß durch den Gegenstand des Patentanspruchs 1 gelöst. Bevorzugte Weiterbildungen, Aspekte und Einzelheiten der Erfindung ergeben sich aus den abhängigen Patentansprüchen, der Beschreibung und den Zeichnungen.

Mit der duftspezifischen Wärmebehandlung ist erstmals eine perfekte und naturgetreue Duftrealisierung möglich.

Die Erfindung ermöglicht es des weiteren, die Qualität der verwendeten Dufteindrücke unverfälscht zu erhalten und unerwünschte Alterungsprozesse und leitungsbedingte Verfälschungen zu vermeiden.

Die Erfindung schafft somit ein Duft-Heiz-System und Leitungssystem für Duftkinos bzw. für Vorführungsvorrichtungen für cinematographische u.a. Aufführungen, welche eine szenengenaue Duftdarbietung ermöglichen.

Gemäß einer bevorzugten Ausgestaltung des erfindungsgemäßen Verfahrens zur Erhöhung der sinnlichen Wahrnehmbarkeit von visuellen und/oder akustischen Darbietungen werden die mit einem Trägergasstrom dem Zuschauer bzw. Zuhörer zugeführten Düfte vor dem Austritt des Trägergases in die dem Zuschauer bzw. Zuhörer umgebende Luft jeweils auf eine duftspezifische Temperatur erwärmt, welche die Entfaltung der Duft- bzw. Aromastoffe gewährleistet. Hierdurch gelingt erstmals die öffentliche Präsentation bestimmter Düfte, da diese bisher durch fehlende Temperaturwerte nicht mehr nach der Erzeugung realisiert werden konnten.

Dabei wird das Trägergas auf die duftspezifische Temperatur erhitzt. Dies kann zweckmäßigerweise dadurch geschehen, daß das Trägergas vor seiner Kontaktierung mit dem jeweiligen Duftaromastoff erhitzt wird. In alternativer Verfahrensführung kann jedoch auch das Trägergas erst nach seiner Kontaktierung mit dem jeweiligen Duftaromastoff erhitzt werden, was vorzugsweise im Leitungssystem geschieht.

Ein Temperaturabfall im Leitungssystem wird sicher vermieden, wenn das Trägergas erst unmittelbar vor seinem Austritt in die den Zuschauer bzw. Zuhörer umgebende Luft auf die duftspezifische Temperatur erhitzt wird.

Um zu vermeiden, daß sich Gerüche in den Leitungen anlegen und mit den jeweils gewünschten Gerüchen überlagern, werden die Leitungen regelmäßig gespült, was vorzugsweise dadurch geschieht, daß das mit Duftaromastoff beladene Trägergas intermittierend mit einem Spülgas zum Zuschauer bzw. Zuhörer geleitet wird. Zweckmäßigerweise erfolgt dabei die Zuführung von duftbeladenem Trägergas und Spülgas in Form von impulsartig aufeinanderfolgenden Intervallen, so daß quasi permanent gespült wird.

Als besonders günstig hat es sich erwiesen, wenn das Trägergas Helium enthält oder aus Helium besteht. Man erhält hierdurch bevorzugt ein Film-Duft-System, bei dem für die Entfaltung der Duftcharakteristika duftspezifisch erwärmtes Helium verwendet wird, welches gleichzeitig einen schnellen Abzug des Duftes am Zuschauersitz gewährleistet. Um Helium zu sparen, werden dabei zweckmäßigerweise alle nicht benutzten Sitze im Duftkino automatisch vom Duftstrom abgeschaltet.

Aus Kostengründen verwendet man als Spülgas bevorzugt Luft, vorzugsweise Druckluft, die zusätzlich erwärmt sein kann.

Speziell für die Duftstoffe eines Film-Duft-Systems ist es wichtig, deren Alterung zu verzögern, was für die Qualität des vom Zuschauer wahrgenommenen Duftes zum Teil sehr wichtig sein kann. Zu diesem Zwecke schlägt die Erfindung vor, daß die Duft- bzw. Aromastoffe bis zur Kontaktierung mit dem Trägergas gekühlt werden, um deren Alterung zu vermeiden. Das Kühlen kann dabei ein Tiefkühlen der Duft- bzw. Aromastoffe sein.

Um der Gefahr zu begegnen, daß sich Duftstoffe beim Transport durch die Leitungswege an den Leitungswänden anlegen, wird zweckmäßigerweise auch das Trägergas und/oder das Spülgas in dem zum Zuschauer bzw. Zuhörer verlaufenden Leitungssystem einer ständigen Wirbelbewegüng, vorzugsweise einer ständigen spiralartigen Rotationsbewegung, unterzogen.

Es wird des weiteren eine Vorrichtung, insbesondere zur Durchführung des vorstehend beschriebenen Verfahrens, beschrieben, in der Mehrfachleitungen zu dem Zuschauer bzw. Zuhörer führen, von denen zeitlich alternierend jeweils zumindest eine für die Duftführung und zumindest eine für die Spülung verwendbar ist. Dies ermöglicht den Aufbau eines Spülsystems für Duftzuleitungen, welches auch während einer Vorstellung spülen kann. Realisiert wird dies bevorzugt zum einen mit einem Zwillingssystem, in welchem ein System Duft zu- und vom Zuschauer nicht gewünschte Düfte ableitet, während das andere System spült und danach wieder verwendet werden kann, sobald sich im ersten System Düfte ansetzen sollten.

Auch mit einem Mehrfachsystem läßt sich dies verwirklichen, indem jeweils die zuletzt benutzten Leitungen gespült und dann auf die anderen verfügbaren Leitungen umgeschaltet wird. Alternativ hierzu ist auch ein Spülsystem möglich, in welchem, soweit gerade keine Dufteinleitung zum Zuschauer stattfindet, jeweils (eventuell ebenfalls erwärmte) Luft durch die Leitungen geführt wird.

Als günstig hat es sich ferner erwiesen, wenn die Vorrichtung eine Kühlvorrichtung enthält, welche den zumindest einen Behälter für die Duft- bzw. Aromastoffe kühlt.

Das Heizsystem für die vorzugsweise in sogenannten Duftkinos verwendeten Duftstoffe muß derart ausgestaltet sein, daß die einzelnen Düfte unterschiedlich, je nach Beschaffenheit und dufttechnischem Erfordernis, erwärmt werden können. Für dieses also duftspezifische Erwärmen kommt vorzugsweise zumindest eine regelbare Heizeinrichtung zur Verwendung, welche das Trägergas erhitzt.

Mit Vorzug wird dabei eine Heizeinrichtung derart vorgesehen, daß sie das Trägergas bereits vor dessen Kontaktierung mit dem zumindest einen Duftaromastoff erhitzt. Alternativ oder zusätzlich hierzu kann je eine Heizeinrichtung benachbart zu der zumindest einen im Zuhörer- bzw. Betrachterbereich befindlichen Duftaustrittsöffnung belegen sein, die vorzugsweise individuell steuerbar ist. Hierdurch werden Wärmeverluste in dem Transportsystem sicher vermieden.

Des weiteren kann eine Heizeinrichtung zwischen dem zumindest einen Behälter für die Duft- bzw. Aromastoffe und der zumindest einen Duftaustrittsöffnung vorgesehen sein.

Zur Verminderung von Duftablagerungen im Leitungssystem enthalten die für die Duftführung verwendbaren Leitungen zumindest an ihrer Innenseite eine Schicht aus einem inerten Material, das vorzugsweise aus Glas oder Keramik besteht. Mit Vorteil sind dabei die für die Duftführungen verwendbaren Leitungen vollständig als Glas- oder Keramikleitungen ausgebildet.

Einem Anlegen der Düfte an den Leitungswandungen wird auch entgegengewirkt, wenn die für die Duftführung verwendbaren Leitungen an den Innenwänden spiralartige oder gleich wirkende Vorsprünge aufweisen oder wenn die für die Duftführung verwendbaren Leitungen spiralförmig geführt sind.

Weitere Einzelheiten der apparativen Ausgestaltung und Verfahrensführung ergeben sich aus der PCT-Anmeldung PCT/EP92/02446 des Anmelders, deren gesamter Inhalt durch die vorliegende Bezugnahme in die Offenbarung der vorliegenden Anmeldung einbezogen wird.

Im folgenden wird die Erfindung unter Bezugnahme auf die Figuren weiter erläutert.

Es zeigen:
- Fig. 1: ein duftspezifisches Heiz- und Regelsystem für Duftkinos
- Fig. 2: einen Filmstreifen mit Wärmesignalen und Regelinformationen
- Fig. 3: ein Kühl- und Trockensystem für Filmduftbehälter
- Fig. 4: einen Längsschnitt durch eine Duft-Zuleitung mit üblichem Strömungsprofil,
- Fig. 5: einen Längsschnitt durch eine Duft-Zuleitung mit innerer Spiraldrehung,
- Fig. 6: einen Längsschnitt durch eine gepufferte und armierte Duft-Glasleitung mit innerer Spiraldrehung,
- Fig. 7: einen Querschnitt durch eine doppelte Zwillingsleitung mit Spülsystem,
- Fig. 8: einen Querschnitt durch ein weiteres Ausführungsbeispiel des Zuleitungssystems aus Fig.7,
- Fig. 9a: einen Querschnitt durch das doppelte Zwillingssystem aus Fig. 8 mit erster Leitung in Spülumschaltung,
- Fig. 9b: einen Querschnitt durch das doppeltes Zwillingssystem aus Fig. 8 mit erster Leitung in Duftumschaltung,

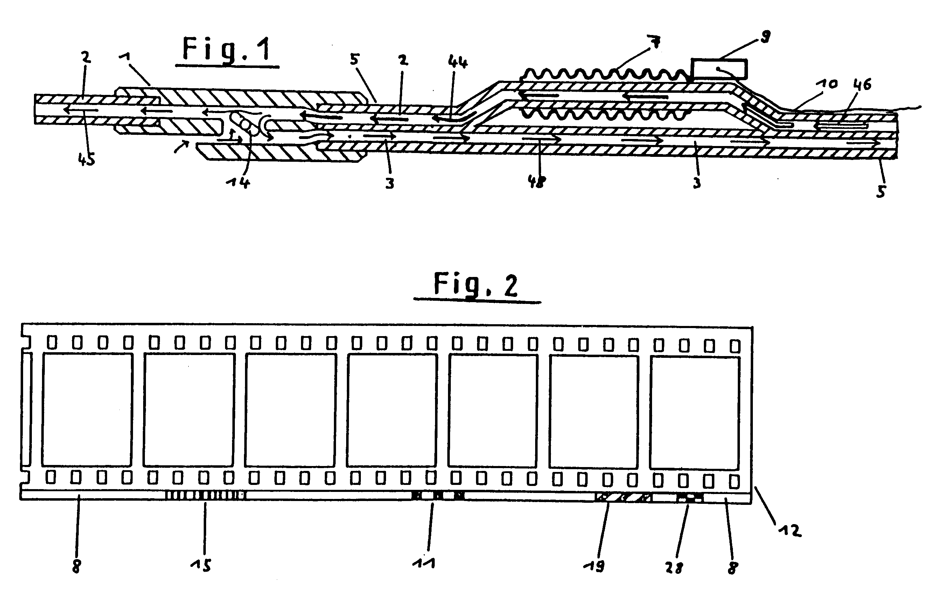

Das in der Fig.1 dargestellte Ausführungsbeispiel der Erfindung zeigt ein Duftheiz-und Regelsystem für Dufteinspielungen bei Filmvorführungen, etc., welches zunächst aus einer Regeleinheit 1 mit einer Zwillingsleitung 5 besteht, die sich wiederum aus einer Duftzuleitung 2 und einer Rückleitung 3 zusammensetzt, wie dies in den einbezogenen Unterlagen der Anmeldung PCT/EP92/02446 vorgesehen ist.
Die Regeleinheit 1 befindet sich auch in dem vorliegenden Ausführungsbeispiel der Erfindung unmittelbar am Kino-Sitzplatz, wobei sich hier die eingespielten, bedufteten Luftmengen im Mikrobereich unterhalb jeglicher Klimatechnik bewegen und zwar vorzugsweise zwischen 0,2 und 0,0002 Liter pro Sekunde.

Die Regeleinheit 1 dient hier dem einzelnen Kinobesucher dazu, die Intensität der über die Duftzuleitung 2 passend zu Filmszenen eingeleiteten Dufteindrücke über den Leitungsregler 14 nach seinem eigenen Empfinden vor-einzustellen (preset). Hierbei wird dann die überflüssige Duft-Luft mit dem Leitungsregler 14 in die Rückleitung 3 geführt.

In der o.g. einbezogenen Anmeldung wurde aus physikalischen Gründen bereits vorgeschlagen, die an dem einzelnen Sitzplatz abgegebenen Düfte 3-4 Grad über der Lufttemperatur im Kino zu erwärmen. Dies sollte dazu führen, das spezifische Gewicht des austretenden Duft/Luft-Gemisches zu verringern, um zur Vermeidung von Überlagerungen der Düfte mehrerer Szenen den sofortigen Abzug des jeweils zuletzt abgegebenen Duftes zur Kinodecke bzw. Klimaanlage hin zu erreichen.

In der vorliegenden Anmeldung wird diese Trennung der szenischen Düfte bedeutend wirkungsvoller durch die Beimischung oder die reine Verwendung von Helium als Duft-Transportmittel erreicht, wie dies weiter unten näher beschrieben wird.

Abgesehen von den physikalischen Eigenschaften bringt eine bestimmte Art der Erwärmung der abgegebenen Duftmengen, sowohl über Luft, Helium oder sonstigen Transportmedien, jedoch einen anderen, sehr bedeutsamen, aromatechnischen Aspekt mit.

Für die tatsächliche Faszination der bei einer Filmvorführung wahrnehmbaren Duftbegleitung ist es bei nicht wenigen Düften und Aromastoffen von entscheidender Bedeutung, wie weit es gelingt, deren gesamtes Duftspektrum perfekt herzustellen.

Viele Duftstoffe entfalten nun ihre wichtigsten Duftcharakteristika nur bei gewissen Temperatureinflüssen. Häufig ist die Wahrnehmbarkeit bzw. Wiedererkennbarkeit von Duft- und Aromastoffen ohne bestimmte Temperatureinflüsse praktisch gar nicht realisierbar, ein Problem mit dem Dufthersteller sehr häufig zu kämpfen haben, da auf die Temperatur eines angewendeten Duftes oder Parfüms i.d.R. keinerlei Einflußmöglichkeit seitens der Hersteller besteht.
So kommt es vor, daß sich bestimmte Duftmerkmale unter gewissen, idealen Temperaturbedingungen herstellen lassen, die dann aber im konkreten Anwendungsfall nicht mehr nachvollzogen werden können.

Bei den hier vorliegenden, filmisch gesteuerten und wie bereits angedeutet, vorzugsweise mit Mikromengen von Helium transportierten Düften, ist es nun erstmals realisierbar, deren ideales Temperaturprofil gleich mitzuliefern und sie dadurch dem Konsumenten überhaupt zum erstenmal erst zugänglich zu machen.

Aus diesem Grund befindet sich in der Fig.1 an der Duftzuleitung 2 vor der Regeleinheit 1 ein Heizsystem 7, welches über eine Heizungs-Steuerung 9 geregelt wird. Die Heizungs-Steuerung 9 wird dabei über ein Steuerkabel 10 mit einem, hier nicht dargestellten, Impulsabtaster verbunden.

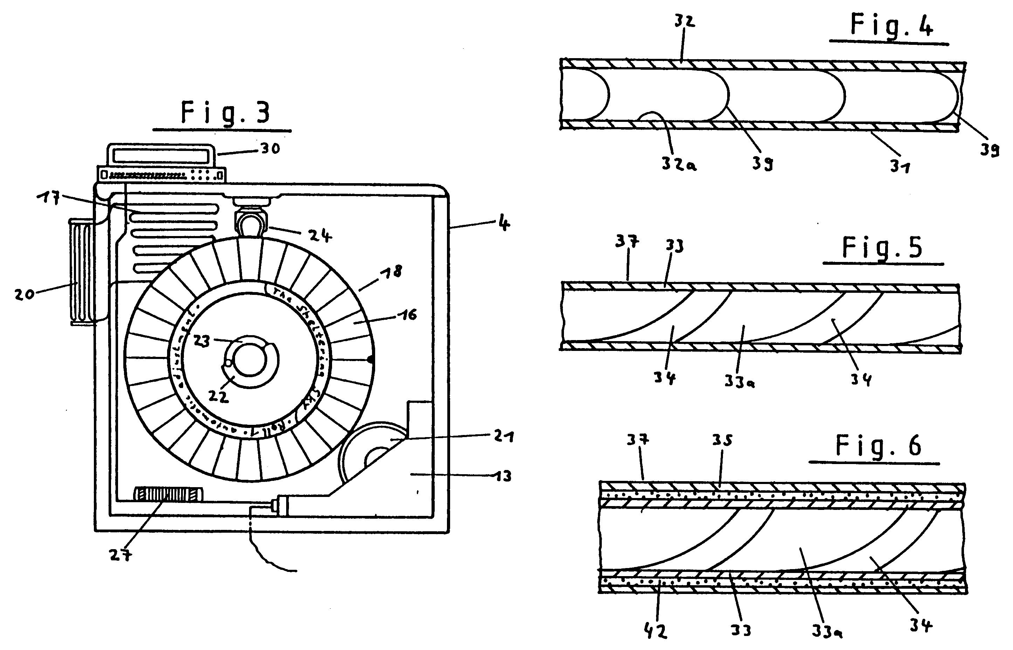

Der Impulsabtaster liest während der Filmvorführung Informationen über den richtigen Heizwert eines Duftes von einer Signalspur 8 des Filmmaterials 12 ab. (Fig.2)
Da die optimalen Temperaturwerte von Duft zu Duft sehr verschieden sein können, werden die entsprechenden, duftspezifischen Temperatur-Signale 15, die das Heizsystem 7 ansteuern, ebenfalls auf der Signalspur 8 des Films angeordnet (Fig.2).

Je nach Ausführung kann das Aufheizen des Heizsystemes 7 auf den angesteuerten Heizwert hin einige, z.B. 3 Sekunden dauern. Aus diesem Grund wird das zugeordenete Temperatur-Signal 15 entsprechend früher auf der Signalspur 8 des Filmmaterials 12 (Fig.2) angebracht, so daß wieder eine Zeitgleichheit zwischen zugeordnetem Temperaturwert mit dem entsprechenden Duft und der zugehörigen Filmszene erreicht wird.

Um den Aufheizvorgang zeitlich kurz zu halten, wird nun die Oberfläche der Zuleitung 2 im Bereich des Heizsystems 7 etwas vergrößert, z.ß. durch eine in diesem Teilstück flach ausgeführte Leitungsform, wodurch auch umgekehrt, bei aufeinander folgenden, niedrigeren Heizwerten eine schnellere Abkühlung des Heizsystems 7 erreicht wird.

Eine Aufheizung kann einen betreffenden Duftwert, unabhängig von seinen sonstigen Eigenschaftsverbesserungen, allgemein verstärken. Daher kann das am Zuschauersitz befindliche Heizsystem 7 bei einem weiteren, nicht dargestellten Ausführungsbeispiel auch teilweise durch den ohnehin für die Duft-Intensität zuständigen Leitungsregler 14 im Niveau der Heizstärke beeinflusst werden.

Bei anderen, hier nicht dargestellten Ausführungsbeispielen der Erfindung kann das duftspezifisch arbeitende Heizsystem 7 auch an weiteren Punkten des Duftleitungssystems eingebaut werden.
Beispielsweise ist ein Einbau am Beginn einer Sammelleitung für 10 Sitzplätze und anderen Punkten des Verteilersystems möglich.

Sofern das Heizsystem dabei direkt hinter dem hier nicht dargestellten, zentralen Dufteinleitungssystem liegt, kommt die ganze Duftkino-Anlage mit nur einem Heizsystem aus, wobei die Heizwerte zum Ausgleich der Leitungsverluste höher liegen müssen, je weiter das Heizsystem vom einzelnen Zuschauersitz entfernt ist.

Bei anderen, im wesentlichen baugleichen Ausführungsbeispielen der Erfindung wird als Transportmedium, statt minimalisierter Luftmengen, Helium verwendet, was zu einem schnellen Verschwinden der abgegebenen Dufteinspielungen führt, da Helium etwa sieben mal leichter als Luft ist.

Der besondere Vorteil hierbei liegt darin, daß ein sehr schneller Abzug von ausgebrachten Düften schon allein durch den Eigenauftrieb des Transportmediums selbst erfolgt, ohne daß mit irgendwelchen störenden Gebläsen oder anderem gearbeitet werden muß.
In Verbindung mit den verwendeten, extrem kleinen Gasmengen werden hierdurch Duftüberlagerungen beim Zuschauer zuverlässig vermieden, ohne daß ein Luftzug spürbar wird. Dies ist für die Vermeidung von jeglichen Ablenkungen vom filmischen Geschehen von großer Bedeutung.

Helium ist dabei sowohl gesundheitlich als auch sicherheitstechnisch hervorragend geeignet. Es ist unbrennbar, chemisch völlig reaktionsträge und gesundheitlich vollkommen unbedenklich, da es schon seit Jahrzehnten erfolgreich für die Atemgeräte von Tauchern und die Atemhilfen von Asthmatikern eingesetzt wird.

Die Leitungsinnenflächen und -führungen müssen dabei aufgrund des erheblich leichteren und schnelleren Fließverhaltens von Helium u.U. etwas angepasst werden.

Trotz der sehr kleinen Mengen Helium, welche zum Dufttransport benutzt werden, kann dessen sparsame Verwendung von Vorteil sein.
Um das überflüssige Ausbringen von kostspieligem Helium zu vermeiden, werden in einem nicht gesondert dargestellten Ausführungsbeispiel der Erfindung die Zuschauerplätze, die während der Filmvorstellung nicht besetzt sind, automatisch vom Duftstrom abgeschaltet.
In einer Abwandlung hiervon werden nur die vom Zuschauer per Handknopf oder per Sitzkontakt aktivierten Plätze in Betrieb gesetzt, womit also nur auf den tatsachlich genutzen Zuschauerplätzen Duftstoffe und Helium verbraucht werden.

Für die Faszination der bei einer Filmvorführung wahrnehmbaren Duftbegleitung ist es neben der Aktivierung von vorhandenen Dufteigenschaften auch wesentlich, daß das vorgesehene Duftprofil eines Duftes in seiner jeweils besonderen Eigenart erhalten bleibt.

Für einen glaubwürdigen und unverfälschten Dufteindruck spielt deshalb der Alterungsprozeß eines Duftes bei vielen Düften eine wichtige Rolle.
Um die während des Filmablaufs abzugebenden Duftstoffe, die bis zu ihrer konkreten Anwendung u.U. viele Wochen lang in dem Film-Duftbehälter eines Kinos verbringen, nicht vorzeitig altern zu lassen, wird die Umgebungstemperatur der zu lagernden Düfte nun bei einem weiteren, in Fig.3 dargestellten Ausführungsbeispiel der Erfindung innerhalb des Film-Duftbehälters 4 stark herabgesetzt.

Hierbei befinden sich die zu einzelnen Szenen gehörigen Duftstoffe in Duftträgern 16, die ihrerseits auf einer Duftrolle 18 angeordnet sind. Die Duftrolle 18 ist drehbar auf einem Achslager 22 gelagert und wird durch eine Achsensicherung 23 fixiert. Die zu den Zuschauern geführte Duftleitung hat über den Duftanschluß 24 die Zugriffsmöglichkeit auf alle Duftträger 16 der Duftrolle 18. Der jeweils richtige, zu einer bestimmten Szene gehörige Duft wird dabei durch Duftimpulse 11 angesteuert, die auf der Signalspur 8 des Filmmaterials 12 liegen, und die an die Duftrollensteuerung 13 geleitet werden. Die Duftrollensteuerung 13 wählt dabei über das Steuerungsrad 21 den zu entsprechenden Szenen passenden Duft an.

Zur Herabsetzung der Umgebungstemperatur der auf der Duftrolle 18 gelagerten Düfte wird nun innerhalb des Film-Duftbehälters 4 ein spezielles Trocken-Kühlsystem 17 eingearbeitet.
Das Trocken-Kühlsystem stellt die Umgebungsluft in dem Film-Duftbehälter 4 nun auf einen für alle auf der Duftrolle 18 befindlichen Düfte optimalen Kühl- und Feuchtigkeitswert ein, welcher je nach Duftrolle 18 bzw. Film leicht differieren kann.

Die für jede Duftrolle 18 spezifischen Kühl- und Feuchtigkeitswerte werden dabei über eine nicht dargestellte Informationsspur an der Duftrolle 18 an die Zentralsteuerung 30 mitgeteilt.

Der entsprechende Kühlwert wird nun von der Zentralsteuerung 30 an das Trocken-Kühlsystem 17 geleitet, wobei zunächst die überschüssige Wärme an die äußeren Wärmetauscher 20 fließt. Die bei Kühlungen häufig auftretenden Ansammlungen von Feuchtigkeit, welche die Qualität von manchen Düften herabsetzen kann, wird nun innerhalb des Film-Duftbehälters 4, durch einen Feuchtigkeitsregler 27 kontrolliert, wobei der Feuchtigkeitsregler 27 ebenfalls von der Zentralsteuerung 30 angesteuert wird.

Die Zentralsteuerung 30 steuert gleichzeitig sämtliche Duftbefehle, die über die Duftsteuerung 13 an die Duftrolle 18 gehen, sowie sämtliche Ventil-, Heiz- etc. Befehle, die insgesamt innerhalb des Kinos vorkommen.

Die über den Duftanschluß 24 aus den Duftträgern 16 der Duftrolle 18 in das Leitungssystem eingespeisten Düfte werden nun unter genauesten elektronischen Steuerungen zu den einzelnen Zuschauern geführt und ggf. durch ein Heizsystem aktiviert. Sofern es sich hierbei um ein zentrales Heizsystem unmittelbar hinter der Dufteinleitung am Duftanschloß 24 handelt, kann die hierfür nötige Wärmeenergie u.U. auch durch einen entsprechend angepassten Wärmetauscher des Kühlsystems entnommen werden.

In dem Ausführungsbeispiel einer Duftzuleitung in Fig.4 wird zunächst gezeigt, welche Art von Strömungsprofil sich in einer stark vergrößerten Duftzuleitung 31 ergibt, wie sie in der einbezogenen Anmeldung PCT/EP92/02446 vorgesehen ist.

Diese Duftzuleitung 31 besteht aus einer Leitungswand 32 mit einer geraden Leitungs-Innenwand 32a und dem dabei normalerweise auftretenden Strömungsprofil 39. Es ist dabei deutlich zu sehen, daß die Strömungsgeschwindigkeit der in dieser Leitung transportierten Luft an den Innenwänden 32a der Leitung stark abnimmt.

Dies kann dazu führen, daß sich an den Rändern solcher Leitungen in dem Luftstrom mitgeführte Mikro-Partikel, hier also Duftpartikel, ablagern können, da sie entsprechend der langsamen Randströmung praktisch nicht genügend mitgerissen werden.

In der Folge können sich im Verlaufe einer längeren Duftabgabe unter Umständen so viele Duftstoffe an den Leitungsrändern ablagern, daß anschließend durchfließende Luft auch ohne mitgeführte Duftstoffe, also reine Luft, den Duft der vorhergehenden Duftabgabe(n) annimmt und in unerwünschter Weise über die zugehörige Szene hinaus weiterhin zum Zuschauer führt.

Ebenso könnte sich dieser frühere Duft mit weiteren Dufteinspielungen vermischen und dadurch ein Duftgemisch erzeugen, welches vom Zuschauer u.U. als unangenehm empfunden wird und außerdem zu keiner Szene mehr passen würde. Das Ergebnis wäre somit statt einer Verstärkung des filmischen Erlebens eine Irritation und Ablenkung hiervon.

Um diesem Mangel zu beheben, weist das in der Fig. 5 vergrößert dargestellte Ausführungsbeispiel der Erfindung, welches aus einer Duft-Zuleitung 37 mit einer Leitungswand 33 besteht, besonders ausgestaltete Innenflächen 33a auf.

Die Innenflächen 33a der Leitungswand 33 besitzen dabei längliche Ausnehmungen oder Vorsprünge 34, welche dazu geeignet sind, die darin fließende Luft in eine ständige Drehbewegung zu versetzen. Im vorliegenden Ausführungsbeispiel haben die Ausnehmungen bzw. Vorsprünge 34 die Form einer auf den Innenflächen 33a der Leitungswand 33 leicht nach Innen vorstehenden, durchgehend über die gesamte Leitungslänge hinweg fortgesetzen Spirallinie.

Dadurch, daß die spiralförmig angeordneten Vorsprünge 34 die durchfließende Luft in eine ständige, ebenfalls spiralartige Drehbewegung versetzen, wird die Geschwindigkeit der an den Leitungsrändern verlaufenden Luft erheblich vergrößert und kann dabei je nach Ausführung sogar größer werden, als die Geschwindigkeit der in der Leitungsmitte fließenden Luft.

Hierdurch werden ebenfalls die im Luftstron mitgeführten Duftpartikel in den Randschichten der Strömung stark beschleunigt, wodurch eine Ablagerung der Duftpartikel an den Innenflächen 33a der Leitungswand 33 weitgehend unterbunden wird.
Die Vorsprünge 34, in diesem Fall also eine Spirallinie wird dabei während des Herstellungsprozesses in die Innenfläche 33a der Leitungswand 33 eingearbeitet.
Herstellbar sind derartige Formen auch durch ein während der Herstellung durchgeführtes, leicht torsionsartiges Verdrehen der Leitung insgesamt.

Um zu vermeiden, daß sich in Strömungsrichtung gesehen, jeweils hinter einem Vorsprung 34 nicht beabsichtigte Mikrowirbel bilden und dabei wiederum Duftstoffe ansammeln, werden die Konturen dieser spiralförmigen Vorsprünge 34 relativ weich ausgestaltet.

Abgesehen von der formmäßigen Beschaffenheit der Innenflächen 33a der Leitungswand 33 ist auch die Feinstruktur von deren innerer Oberfläche, sowie auch eine eventuelle chemische Reaktionsbereitschaft dieses Materials für die Vermeidung der Anlagerung von Duftpartikeln wichtig.

Bezüglich der chemischen Reaktionsbereitschaft mit Duftstoffen sind die meisten Kunststoffe reaktionsanfällig und daher trotz der günstigen Verarbeitungsmöglichkeit i.d.R. ungeeignet.
Bei einer vorzugsweisen Ausführung handelt es sich bei dem Material der Leitungswand 33 daher um besonders reaktionsträge Stahlsorten z.B. V4a-Stahl.

Dennoch können auch Rohre aus Stahl und anderen reaktionsträgen Materialien Probleme mit sich bringen. Ist deren Oberfläche bei mikroskopischer Betrachtung z.B. sehr rissig und von vielfältigen, kleinsten Vertiefungen durchzogen, können sich dort leichter Duftstoffe anlagern, als bei sehr glatter Oberfläche.

In einem weiteren, in der Fig. 6 vergrößert dargestellten Ausführungsbeispiel werden daher besonders vorteilhafte Werkstoffe für die Leitungswände 33 der Duftzuleitungen 37 verwendet und zwar bestimmte, relativ elastische Glassorten. Glasleitungen haben bezüglich Mikropartikeln die auch im Mikrobereich glattesten Oberflächen, weshalb eine Anlagerung von Duftstoffen hierbei weitestgehend ausgeschlossen ist.

Auch in diese Glasleitungen können jedoch zusätzlich Strukturen bzw. spiralartige Vorsprünge 34 eingelassen werden, welche die durchfließende Luft bis zu ihrem Austritt am Sitz des Zuschauers in eine ständige Drehbewegung versetzen.

In den besonderen Duftzuleitungen 37 des Ausführungsbeispiels in Fig. 6 wird eine Anlagerung von Duftstoffen somit auf doppelte Weise vermieden:
Die spiralige Grobstruktur der Oberfläche beschleunigt die bewegten Luftmengen in den Randschichten der Strömung so stark, daß eine Verlangsamung der mitgeführten Duftmoleküle mit anschliessender Anlagerungsmöglichkeit am Leitungsrand 33a unterbunden wird, während zusätzlich die glatte Feinstruktur der Innenfläche 33a der Leitungswände 33 die Anlagerung von Duftmolekülen auch unter ungünstigsten Strömungsbedingungen verhindert.

Auch bei elastischen Glassorten kann es von Vorteil sein, diese vor äußeren Belastungen zu schützen, insbesondere falls diese nicht unter dem Fußboden verlegt, sondern oberhalb des Fußbodens als Mikroleitung nachträglich in bestehende Filmtheater eingebaut werden.
Deshalb sind die Leitungswände 33 in dem Ausführungsbeispiel in Fig. 6 zusätzlich in eine elastische Schicht 42 eingebettet, welche ihrerseits in einer verformungsbeständigen Schutzhülse 35 gelagert ist, die z.B. aus Hartkunststoff oder Hartstahl gefertigt wird.

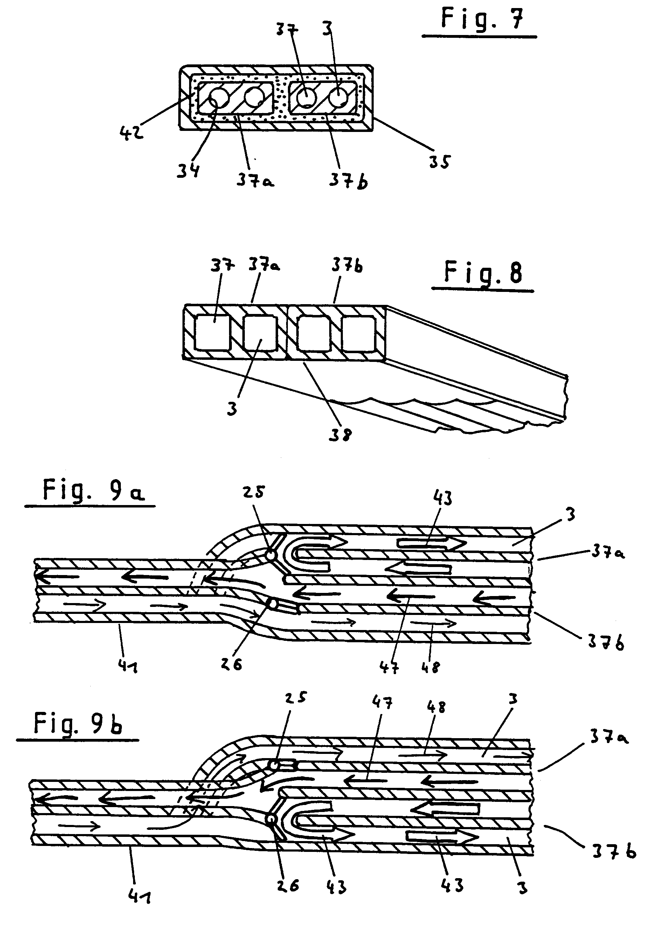

Bei einem weiteren Ausführungsbeispiel der Erfindung nach Fig.7 werden Duftablagerungen am Leitungsrand nicht in erster Linie durch die Oberflächenbeschaffenheit der Leitungen verhindert.

In dieser Ausführung werden für die Duftzuleitung 40 jeweils zwei Zwillingsleitungen 37a und 37b verwendet, wobei beide Leitungen in einer elastischen Masse 42 gelagert sind, die wiederum von der flachen Gesamthülse 36 umhüllt wird.

Bei diesen beiden Zwillingsleitungen wird jeweils nur eine Leitung z.B. die Leitung Nr. 37a für die Duftzuführung benutzt. Nach bestimmten, durch Erfahrungswerte vorgegebenen Intervallen (z.B. nach jeweils 60 Funktionsminuten), die den Anfang auch nur leichtester Duftablagerungen erkennen lassen, kann in der Duftzuleitung 40 von der Zwillingsleitung 37a auf die andere Zwillingsleitung 37b umgeschaltet werden.

Soweit irgendwann beide Zwillingsleitungen 37a und 37b benutzt sind, können die Leitungen nach dem in der ursprünglichen Anmeldung DE-A-4135796.5 beschriebenen Schema gespült werden, indem eine Doppelleitung, z.B. 37a an der Austrittsöffnung zusammengeschlossen wird.

Hierbei wird nun statt Luft eine Reinigungsflüssigkeit in eine der beiden Leitungen der Zwillingsleitung 37b eingebracht, welche die gesamte Leitung bis zum hier nicht dargestellten Zuschauersitz durchfließt, dann in die zweite Leitung überwechselt und anschließend zum hier ebenfalls nicht dargestellten Kompressor zurückfließt. Beide Einzelleitungen der Zwillingsleitung 37b werden hierbei gleichzeitig gereinigt, während zum Trocknen der Leitungen gleich anschließend erwärmte Luft nachfließt.

Soweit es bei dem Ausführungsbeispiel der Erfindung nach den Figuren 7, 8, 9a und 9b abzusehen ist, daß beide Zwillingsleitungen 37a und 37b bereits während einer Filmvorführung verbraucht sein sollten, ist es aufgrund der sehr kleinen Leitungsdurchmesser und dem relativ geringen Druck auch möglich, eine der beiden Zwillingsleitungen ohne merkliche Geräuschentwicklung während einer Filmvorführung zu reinigen während die andere Zwillingsleitung weiterhin für die Duftzuleitung und -ableitung benutzt wird.

Ein derartiger Spülvorgang kann aufgrund der sehr kleinen Volumina in ca. 0,5 bis 3 Minuten durchgeführt werden.

Soweit bei dieser Ausführung eine Leitungsspülung während einer Filmvorführung erfolgen soll, werden die Zwillingsleitungen 37a und 37b im Endbereich am Zuschauersitz vorzugsweise zunächst in einem Funktions-Block 38 zusammengefasst und die Leitungsquerschnitte vorzugsweise von runden in rechteckige Formen verändert (Fig.8).

In den Figuren 9a und 9b des Ausführungsbeispiels aus Fig.8 wird gezeigt, wie das Leitungsendstück funktioniert, sofern eine Leitungsspülung während einer Filmvorführung erfolgen soll. Hierbei wird der Zusammenschluß zweier Leitungen einer Zwillingsleitung 37b, an deren Austrittsöffnung, d.h. also am Leitungs-Endstück im Bereich des Zuschauersitzes mit elektronisch angesteuerten, geräuschlos funktionierenden End-Ventilen 25 oder 26 durchgeführt.

Ist hierbei das Endventil 25 aktiviert und das Endventil 26 deaktiviert, kann die Zwillingsleitung 37b weiterhin für die Duftzu- und Ableitung benutzt werden, während die Zwillingsleitung 37a gespült wird (Fig. 9a) Hierbei setzt sich die Leitung 37b in das Leitungs-Endstück 41 hinein, zum Zuschauer hin fort.

Sobald nun die Zuleitung der Zwillingsleitung 37b Duftanlagerungen befürchten läßt, wird in einer geeigneten Duftpause das Ventil 26 aktiviert und das Ventil 25 deaktiviert. Hierauf wird die Duftzu- und ableitung nun durch die vorher gereinigte Zwillingsleitung 37a zum Leitungsendstück geführt und die Zwillingsleitung 37b gespült.

Geräuschlose Ventilumschaltungen können hierbei mit verschiedenen Ventiltypen und -anordnungen z.B. auch mit Schiebe- und Schleusen-Ventilen erreicht werden. oder z.B. mit besonders langsam umschaltenden Ventilen, die zusätzlich gedämpft sein können.

Alle End-Ventile 25 und 26 zusammengenommen werden dabei zentral, z.B. vom Filmvorführer-Raum aus über Funk oder über eine kleine, elektronische Leitung, die in das Duftleitsystem integriert sein kann, angesteuert.

Eine geräuschlose Ventilausführung bewirkt hier, daß bei einer gleichzeitigen Umschaltung aller Ventile, z.B. bei 500 Sitzen somit 500 Ventilen, keine die Filmvorführung beeinträchtigenden Störungen auftreten.
Soweit sich bei der gleichzeitigen Umschaltung sehr vieler Ventile kleine Geräusche nicht ganz vermeiden lassen, ist es auch möglich, die Umschaltung Segmentweise, also zuerst bei z.B. 20% der Sitze durchzuführen, dann bei den nächsten 20%, usw.

Eine weitere Maßnahme, um auch kleinste Geräusche bei der Ventilumschaltung oder auch bei dem anschließenden Spülvorgang vollständig zu vermeiden, wird erreicht, indem die z.B. 1 Minute dauernde Umschaltung und Spülung einer Leitung genau während einer der lautstärkeintensiveren Sequenzen eines Films durchgeführt wird.

Hierbei überdeckt dann die filmbegleitende Geräuschkulisse eventuell auftretende Geräusche. Das genau nach dem Beginn einer geräuschintensiven Filmsequenz zu setzende Signal 19 zur Ansteuerung der Schalt- und Spülphase befindet sich dabei vorzugsweise auf der Signalspur 8 des Films, wo sich auch die anderen Signale zur Duftansteuerung befinden.

Eine weitere Maßnahme, um ein eventuelles Ansammeln von Duftstoffen in den Leitungen zu vermeiden, läßt sich erreichen, indem die Leitungen in allen Phasen des Films, in denen keine Duftstoffe zum Zuschauer transportiert werden, mit normaler und eventuell leicht erwärmter Luft gespült werden.
Hierzu befindet sich unmittelbar nach dem Duftbehälter, woraus die Duftstoffe in die Leitungen eingeleitet werden, also innerhalb des Filmvorführerraumes, ein hier nicht dargestelltes Umschaltventil.

Das Umschaltventil wird dabei nun Betrieb gesetzt, sobald für einen bestimmten Zeitintervall keine Dufteinspeisungen in Betrieb gesetzt wurden und wird abgeschaltet, soweit die nächste Dufteinspeisung einsetzt.

## Patentansprüche

1. Verfahren zur Erhöhung der sinnlichen Wahrnehmbarkeit von visuellen und/oder akustischen Darbietungen, insbesondere in Kinos, Theatern, Konzert- und Vortragssälen sowie bei Dia-Vorträgen, Videos, Fernsehsendungen, Hörspielen und dergleichen, bei dem Zuschauern bzw. Zuhörern synchron zur Darbietung von bestimmten visuellen und/oder akustischen Reizen dazu passende Düfte zugeführt werden, wobei die mit einem Trägergasstrom dem Zuschauer bzw. Zuhörer zugeführten Düfte vor dem Austritt des Trägergases in die den Zuschauer bzw. Zuhörer umgebende Luft jeweils auf eine duftspezifische Temperatur erwärmt werden, welche die Entfaltung der Duft- bzw. Aromastoffe gewährleistet, dadurch gekennzeichnet, daß das Trägergas auf die duftspezifische Temperatur erhitzt wird.

2. Verfahren nach Anspruch 1, dadurch gekennzeichnet, daß das Trägergas vor seiner Kontaktierung mit dem jeweiligen Duftaromastoff erhitzt wird.

3. Verfahren nach Anspruch 1 oder 2, dadurch gekennzeichnet, daß das Trägergas nach seiner Kontaktierung mit dem jeweiligen Duftaromastoff erhitzt wird.

4. Verfahren nach Anspruch 3, dadurch gekennzeichnet, daß das Trägergas im Leitungssystem erhitzt wird.

5. Verfahren nach Anspruch 3 oder 4, dadurch gekennzeichnet, daß das Trägergas unmittelbar vor seinem Austritt in die den Zuschauer bzw. Zuhörer umgebende Luft auf die duftspezifische Temperatur erhitzt wird.

6. Verfahren nach einem der vorstehenden Ansprüche, dadurch gekennzeichnet, daß das mit Duftaromastoff beladene Trägergas intermittierend mit einem Spülgas zum Zuschauer bzw. Zuhörer geleitet wird.

7. Verfahren nach Anspruch 6, dadurch gekennzeichnet, daß die Zuführung von duftbeladenem Trägergas und Spülgas in Form von impulsartig aufeinanderfolgenden Intervallen erfolgt.

8. Verfahren nach einem der vorstehenden Ansprüche, dadurch gekennzeichnet, daß das Trägergas Helium enthält.

9. Verfahren nach Anspruch 8, dadurch gekennzeichnet, daß als Trägergas Helium verwendet wird.

10. Verfahren nach einem der Ansprüche 6 bis 9, dadurch gekennzeichnet, daß als Spülgas Luft, vorzugsweise Druckluft, verwendet wird.

11. Verfahren nach einem der vorstehenden Ansprüche, dadurch gekennzeichnet, daß die Duft- bzw. Aromastoffe bis zur Kontaktierung mit dem Trägergas gekühlt werden, um deren Alterung zu vermeiden.

12. Verfahren nach Anspruch 11, gekennzeichnet durch ein Tiefkühlen der Duft- bzw. Aromastoffe.

13. Verfahren nach einem der vorstehenden Ansprüche, dadurch gekennzeichnet, daß das Trägergas und/oder das Spülgas in dem zum Zuschauer bzw. Zuhörer verlaufenden Leitungssystem einer Wirbelbewegung unterzogen wird.

14. Verfahren nach einem der vorstehenden Ansprüche, dadurch gekennzeichnet, daß das Trägergas und/oder das Spülgas in dem zum Zuschauer bzw. Zuhörer führenden Leitungssystem in eine vorzugsweise spiralartige Rotationsbewegung versetzt wird.

15. Verfahren nach Anspruch 1, dadurch gekennzeichnet, daß eingespielte, beduftete Luftmengen sich im Mikrobereich, vorzugsweise zwischen 0,2 und 0,0002 Liter pro Sekunde bewegen.

16. Verfahren nach Anspruch 1, dadurch gekennzeichnet, daß der richtige Heizwert eines Dufts von einer Signalspur (8) eines Filmmaterials (12) abgelesen wird.

17. Verfahren nach einem der Ansprüche 8 oder 9, dadurch gekennzeichnet, daß Zuschauerplätze, die während der Filmvorstellung nicht besetzt sind, automatisch vom Duftstrom abgeschaltet werden.

18. Verfahren nach einem der Ansprüche 8 oder 9, dadurch gekennzeichnet, daß nur vom Zuschauer per Handknopf oder per Sitzkontakt aktivierte Plätze in Betrieb gesetzt werden.

## Claims

1. Process for intensifying sensorial perception of visual and/or acoustic presentations, particularly in cinemas, theaters, concert and conference halls, as well as during slide and video shows, television and radio broadcasts and the like, wherein the audience is supplied with respectively suitable scents in synchronism with the presentation of specific visual and/or acoustic stimuli, and the scents supplied to the audience via a carrier gas stream are respectively heated to a scent-specific temperature before the carrier gas is discharged into the ambient air around the audience, said scent-specific temperature ensuring the development of the scents or aromas, characterized in that the carrier gas is heated to the scent-specific temperature.

2. Process according to claim 1, characterized in that the carrier gas is heated before it gets into contact with the respective aromatic substance.

3. Process according to claim 1 or 2, characterized in that the carrier gas is heated after it has come into contact with the respective aromatic substance.

4. Process according to claim 3, characterized in that the carrier gas is heated in the conduit system.

5. Process according to claim 3 or 4, characterized in that the carrier gas is heated to the scent-specific temperature directly before it is discharged into the ambient air around the audience.

6. Process according to any one of the preceding claims, characterized in that the carrier gas charged with the aroma is supplied intermittently with a purging gas to the audience.

7. Process according to claim 6, characterized in that the carrier gas charged with aroma and the purging gas are supplied in pulse-like successive intervals.

8. Process according to any one of the preceding claims, characterized in that the carrier gas contains helium.

9. Process according to claim 8, characterized in that helium is used as carrier gas.

10. Process according to any one of claims 6 to 9, characterized in that air, preferably compressed air, is used as purging gas.

11. Process according to any one of the preceding claims, characterized in that the scents or aromas are cooled in order to prevent ageing until they come into contact with the carrier gas.

12. Process according to claim 11, characterized in that the scents or aromas are deep-cooled.

13. Process according to any one of the preceding claims, characterized in that the carrier gas and/or the purging gas is/are subjected to a whirling motion in the conduit system leading to the audience.

14. Process according to any one of the preceding claims, characterized in that a preferably spiral rotary motion is imparted on the carrier gas and/or the purging gas in the conduit system leading to the audience.

15. Process according to claim 1, characterized in that scent-charged quantities of air introduced are in the micro-range, preferably between 0.2 and 0.0002 l/sec.

16. Process according to claim 1, characterized in that the appropriate thermal value of a scent is read from a signal track (8) of a film stock (12).

17. Process according to one of claims 8 or 9, characterized in that seats that are not occupied during the showing of a movie are automatically disconnected from the scent stream.

18. Process according to one of claims 8 or 9, characterized in that only seats activated by the audience by pushbutton or upon seat contact are operated.

## Revendications

1. Procédé d'augmentation de la perceptibilité sensorielle de représentations visuelles et/ou acoustiques, en particulier de cinémas, théâtres, salles de concert et salles de conférences ainsi que lors de conférences avec projection de diapositives, vidéos, émissions télévisuelles, pièces radiophoniques et autres, chez le spectateur ou l'auditeur, des parfums adaptés à cet effet sont introduits de façon synchrone par rapport à la représentation de certaines stimulations visuelles et/ou acoustiques, les parfums amenés jusqu'au spectateur ou jusqu'à l'auditeur avec un courant de gaz porteur étant chaque fois réchauffés avant la sortie du gaz porteur dans l'air environnant le spectateur ou l'auditeur à une température spécifique qui garantit la diffusion des parfums ou arômes, caractérisé en ce que le gaz porteur est échauffé à la température spécifique au parfum.

2. Procédé selon la revendication 1, caractérisé en ce que le gaz porteur est échauffé avant d'entrer en contact avec chaque parfum.

3. Procédé selon la revendication 1 ou 2, caractérisé en ce que le gaz porteur est échauffé après être entré en contact avec chaque parfum.

4. Procédé selon la revendication 3, caractérisé en ce que le gaz porteur est échauffé dans le système de conduite.

5. Procédé selon la revendication 3 ou 4, caractérisé en ce que le gaz porteur est immédiatement échauffé à la température spécifique au parfum avant sa sortie dans l'air environnant le spectateur ou l'auditeur.

6. Procédé selon une des revendications précédentes, caractérisé en ce que le gaz porteur chargé avec le courant de parfum est dirigé de façon discontinue avec un gaz de circulation vers le spectateur ou l'auditeur.

7. Procédé selon la revendication 6, caractérisé en ce que l'introduction de gaz porteur chargé de parfum et de gaz de circulation a lieu à intervalles successifs par impulsions.

8. Procédé selon une des revendications précédentes, caractérisé en ce que le gaz porteur contient de l'hélium.

9. Procédé selon la revendication 8, caractérisé en ce que l'hélium est utilisé comme gaz porteur.

10. Procédé selon une des revendications 6 à 9, caractérisé en ce que l'air, de préférence l'air comprimé, est utilisé comme gaz de circulation.

11. Procédé selon une des revendications précédentes, caractérisé en ce que les parfums et arômes sont refroidis jusqu'à l'entrée en contact avec le gaz porteur pour éviter leur altération.

12. Procédé selon la revendication 11, caractérisé par une réfrigération des parfums et des arômes.

13. Procédé selon une des revendications précédentes, caractérisé en ce que le gaz porteur et/ou le gaz de circulation est soumis à un mouvement tourbillonnaire dans le système de conduite allant vers le spectateur ou l'auditeur.

14. Procédé selon une des revendications précédentes, caractérisé en ce que le gaz porteur et/ou le gaz de circulation est déplacé dans un mouvement de rotation, de préférence en spirale dans le système de conduite dirigé vers le spectateur ou l'auditeur.

15. Procédé selon la revendication 1, caractérisé en ce que des volumes d'air parfumés équilibrés se déplacent dans des micro quantités, de préférence entre 0,2 et 0,0002 litre seconde.

16. Procédé selon la revendication 1, caractérisé en ce que la valeur calorifique exacte d'un parfum est lue par une piste signalétique (8) d'un film (12).

17. Procédé selon la revendication 8 ou 9, caractérisé en ce que les places des spectateurs qui sont inoccupées pendant la diffusion du film, sont automatiquement coupées du débit de parfum.

18. Procédé selon la revendication 8 ou 9, caractérisé en ce que seules les places activées par le spectateur par l'intermédiaire d'une manette ou par contact lorsque le spectateur est assis, sont mises en service.
